# EUROPEAN PATENT SPECIFICATION

(11) **EP 3 948 320 B1**
(45) Date of publication and mention of the grant of the patent: **14.06.2023**
(21) Application number: 20713322.4
(22) Date of filing: 27.03.2020
(51) Int. Cl.: G01R 33/20, A61B 5/055

(54) **TWO-DIMENSIONAL DISPLAY FOR MAGNETIC RESONANCE IMAGING**
ZWEIDIMENSIONALE ANZEIGE FÜR DIE MAGNETRESONANZBILDGEBUNG
AFFICHAGE BIDIMENSIONNEL POUR IMAGERIE PAR RÉSONANCE MAGNÉTIQUE

(30) Priority: 01.04.2019 EP 19166568
(43) Date of publication of application: 09.02.2022
(73) Proprietor: Koninklijke Philips N.V., 5656 AG Eindhoven (NL)
(72) Inventor: FORTHMANN, Peter, 5656 AE Eindhoven (NL); KRUEGER, Sascha, 5656 AE Eindhoven (NL); WUELBERN, Jan, Hendrik, 5656 AE Eindhoven (NL)
(74) Representative: Philips Intellectual Property & Standards
(86) International application number: PCT/EP2020/058851
(87) International publication number: WO 2020/201154

(56) References cited:
- CN-A- 103 860 179
- CN-U- 201 920 723
- US-A1- 2005 283 068

## Description

### FIELD OF THE INVENTION

The invention relates to magnetic resonance imaging, in particular to the construction of magnetic resonance imaging systems.

### BACKGROUND OF THE INVENTION

A large static magnetic field is used by Magnetic Resonance Imaging (MRI) scanners to align the nuclear spins of atoms as part of the procedure for producing images within the body of a patient. This large static magnetic field is referred to as the BO field or the main magnetic field. Time dependent magnetic gradient fields and radio frequency (RF) fields are used to perform a spatially dependent manipulation the orientation of the spins. Electronic components and conductive components can interact with the magnetic and radio frequency fields.

United States patent publication US 2014/125337 A1 discloses a magnetic resonance imaging (MRI) apparatus which includes a housing which has a bore to which a magnetic field for use in an MRI scan is applied, a moving table on which an inspection target may be placed and that enters the bore of the housing, a projector which projects an image onto an inner wall that forms the bore of the housing, and a controller which controls the projection unit and transmits a video signal to the projector. CN201920723, CN103860179 and US2005283068 disclose digital video projection displays for magnetic resonance imaging purposes.

### SUMMARY OF THE INVENTION

The invention provides for a magnetic resonance imaging magnet assembly, and a magnetic resonance imaging system in the independent claims. Embodiments are given in the dependent claims.

For various applications which seek to increase patient comfort and experience, high-resolution optical displays (two-dimensional display) inside the MRI bore have a tremendous potential. When integrating a screen with display control electronics into the MR bore, electromagnetic interference can become a challenge. Embodiments of the invention may provide for an improved display by using an optical waveguide bundle which couples to a two-dimensional display. The two-dimensional display has diffusors which each couple to at least on optical waveguide. Each of these diffusors forms a pixel in the display. The diffusor provides for a display which is very compact and which may be viewed from a large angular range.

In one aspect the invention provides for a magnetic resonance imaging magnet assembly configured for supporting a subject within an imaging zone. The magnetic resonance imaging magnet assembly comprises a magnetic resonance imaging magnet. The magnetic resonance imaging magnet is configured for generating a main magnetic field with an imaging zone. An imaging zone as used herein encompasses a region where the magnetic field has a high enough value and is uniform enough to perform magnetic resonance imaging. The magnetic resonance imaging magnet assembly is configured for supporting at least a portion of the subject within the imaging zone.

The magnetic resonance imaging magnet assembly further comprises an optical image generator configured for generating a two-dimensional image. The magnetic resonance imaging magnet assembly further comprises an optical waveguide bundle configured for coupling to the optical image generator. The magnetic resonance imaging magnet assembly further comprises a two-dimensional display comprising pixels. Each of the pixels comprises a diffusor, wherein the diffusor is a diffusor plate.

A diffuser as used herein encompasses an optical structure which is used to make the illumination of the pixel uniform or within a predetermined uniformity and/or used to define or control the size of a pixel. The diffuser is a diffuser plate or may also be formed within an end point or end tip of the optical waveguide bundle. Each of the pixels is optically coupled to at least one optical waveguide selected from the optical waveguide bundle. The at least one optical waveguide of each of the pixels is configured for illuminating the diffuser. The optical waveguide bundle and the two-dimensional display are configured for displaying the two-dimensional image.

The optical waveguide bundle couples to the optical image generator and then this is then displayed on the two-dimensional display which comprises the pixels. This may be beneficial because the use of diffusers enables the construction of a display which is compatible with magnetic resonance imaging and also can be viewed from a variety of angles. This makes it less critical in the placement of the two-dimensional display with respect to a subject. It also enables the subject to view the two-dimensional display with less fatigue and with less effort.

The optical image generator for example is a screen, projector or other type of display. The use of the optical waveguide bundle enables the optical image generator to be removed from the high field regions of the main magnetic field.

The diffuser is integrated into the individual waveguides or may in some examples be a separate diffuser plate to which the individual optical waveguides are coupled.

In another embodiment the magnetic resonance imaging magnet assembly further comprises a subject support. The optical waveguide bundle is integrated into the subject support. This example may for example be beneficial because it enables the image to be brought into the magnetic resonance imaging system where the subject can view it. Placing the optical waveguide bundle in the support may be beneficial or useful because the optical waveguide bundle will very likely not interfere with the magnetic resonance imaging protocol.

In another embodiment the subject support comprises a support arch. The two-dimensional display is attached to the support arch. This embodiment may be beneficial because as the support arch is attached to the subject support as the subject support may be moved into the magnetic resonance imaging system the position of the two-dimensional display will therefore have a constant position with respect to the subject. This for example may enable the positioning or alignment of the two-dimensional display outside of the bore of the magnetic resonance imaging magnet.

In another embodiment the magnetic resonance imaging magnet assembly comprises a gradient coil assembly. The magnetic resonance imaging magnet assembly comprises a magnet cover encasing the magnetic resonance imaging magnet and the gradient coil assembly. The two-dimensional display may be in one embodiment integrated into the magnet cover and attached to the magnet cover.

For example, the optical waveguide bundle may be formed or manufactured as a part of the magnet cover. In a different embodiment the optical waveguide bundle is attached to the magnet cover. For example, the optical waveguide bundle may be manufactured and then later attached or glued or taped to the magnet cover. In a different variant of the embodiment the optical waveguide bundle is between the gradient coil assembly and the magnet cover. For example, if the optical waveguide bundle is a bundle of optical fibers it may simply be placed or spread in between the two and may also not need to be attached or formed into the magnet cover.

In another embodiment the magnetic resonance imaging magnet is a cylindrical magnet with a bore for receiving the subject. The two-dimensional display is within the bore. For example, the two-dimensional display may be attached within the bore of the magnet. This may be beneficial because the use of the optical waveguide bundle may enable a very compact two-dimensional display to be permanently mounted within the bore of the magnet.

In another embodiment the optical image generator is attached to the magnetic resonance imaging magnet assembly. The optical image generator is outside of the bore.

In another embodiment the optical waveguide bundle is formed from multiple optical fibers. This may be a very convenient and economical means of forming the optical waveguide bundle.

In another embodiment the optical waveguide bundle is a three-dimensional printed optical waveguide bundle or a waveguide bundle formed from lithographically structured foils. This embodiment may be beneficial because it may be very conveniently formed into another component of the magnetic resonance imaging magnet assembly such as a cover or it may be built into another component as it is manufactured.

In another embodiment the optical waveguides of the optical waveguide bundle are configured for forming an optical coupling surface that abuts the diffuser of each voxel. For example, if they are fibers, they may have their end point mounted flush with a diffuser or diffuser plate.

In another embodiment the optical waveguide bundle is configured and manufactured such that the diffuser forms an end surface of the optical waveguide. For example, the end of the waveguide in the optical waveguide bundle may be frosted so that they diffuse light. This embodiment may also involve the broadening of the optical waveguide bundle so to control the size of the particular pixel. This may be a particularly beneficial embodiment for example when the optical waveguide bundle is 3D printed or formed from lithographically structured foils. This may enable the forming of the complete diffuser and optical waveguide bundle in one step.

In another embodiment the optical waveguides of the optical waveguide bundle comprise a reflective end surface. For example, the end of each of the waveguides may be polished flat or even silver. The optical waveguides of the optical waveguide bundle comprise a length extension. The optical waveguides of the optical waveguide bundle are configured to couple the diffuser using the reflected end surface. This embodiment may be beneficial because it may enable reducing the size or thickness of the combination of the optical waveguide bundle and the two-dimensional display.

In another aspect the invention provides for a magnetic resonance imaging system that comprises a magnetic resonance imaging magnet assembly according to an embodiment. The magnetic resonance imaging system further comprises a memory for storing machine-executable instructions and pulse sequence commands configured for controlling the magnetic resonance imaging system to acquire magnetic resonance imaging data. The magnetic resonance imaging system further comprises a processor configured for controlling the magnetic resonance imaging system.

The execution of the machine-executable instructions causes the processor to acquire the magnetic resonance imaging data by controlling the magnetic resonance imaging system with the pulse sequence commands. Execution of the machine-executable instructions further causes the processor to control the optical image generator to generate the two-dimensional image during the acquisition of the magnetic resonance imaging data. This embodiment may be beneficial because it provides a means for efficiently providing the two-dimensional image to the subject during the acquisition of the magnetic resonance imaging data without having a detrimental effect on this acquisition.

In another embodiment the magnetic resonance imaging system further comprises a subject motion detection system configured for acquiring the subject motion during the acquisition of the magnetic resonance imaging data. Execution of the machine-executable instructions further causes the processor to control the subject motion detection system to acquire the subject motion data during the acquisition of the magnetic resonance imaging data. Execution of the machine-executable instructions further causes the processor to control the optical image indicator to render a motion feedback indicator using the subject motion data.

For example, the optical image indicator could be an image or display which is used to display a symbol or figure which represents the subject motion data. During some magnetic resonance imaging protocols, it is beneficial that the subject holds his or her breath. The optical image indicator can be used to indicate a breathing phase that the subject should remain in. This may provide feedback and help the subject concentrate to hold his or her breath. In other examples the subject may have a tendency to move and the optical image indicator may provide a diagram of the subject's body position. This feedback may help the subject from moving.

In another embodiment the subject motion detection system comprises a body position sensor.

In another embodiment the motion detection system comprises a camera system.

In another embodiment the motion detection system comprises a respiration tube.

In another embodiment the motion detection system comprises a respiration monitor belt.

In another embodiment the subject motion detection system comprises a magnetic resonance imaging navigator. For example, the motion detection system could be the magnetic resonance imaging itself when it is acquiring a navigator which is used to measure the position of the subject.

In another embodiment the optical image indicator is configured for displaying any one of the following: a breath hold indicator, a breathing state of the subject, a body position of the subject and combinations thereof.

In another embodiment execution of the machine-executable instructions causes the processor to control the optical image generator to render a chosen color pattern.

In another embodiment execution of the machine-executable instructions further cause the processor to render a chosen color gradient.

In another embodiment execution of the machine-executable instructions further cause the processor to render a chosen brightness gradient.

The rendering of the chosen color pallet, the chosen color gradient, and the chosen brightness gradient may be used for controlling the color and/or lighting within the magnetic resonance imaging system. This for example may provide a calming or soothing effect on the subject and it may also be useful in controlling the mood of the subject.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A `computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a processor. `Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Magnetic Resonance (MR) imaging data is defined herein as being the recorded measurements of radio frequency signals emitted by atomic spins using the antenna of a Magnetic resonance apparatus during a magnetic resonance imaging scan. A Magnetic Resonance Imaging (MRI) image or MR image is defined herein as being the reconstructed two- or three-dimensional visualization of anatomic data contained within the magnetic resonance imaging data. This visualization can be performed using a computer.

### BRIEF DESCRIPTION OF THE DRAWINGS

In the following preferred embodiments of the invention will be described, by way of example only, and with reference to the drawings in which:
Fig. 1 illustrates an example of a magnetic resonance imaging system;
Fig. 2 illustrates a further example of a magnetic resonance imaging system:
Fig. 3 shows a flow chart which illustrates a method of operating either the magnetic resonance imaging system of Fig. 1 or Fig. 2;
Fig. 4 illustrates an example of a two-dimensional image which renders an example of a motion feedback indicator;
Fig. 5 illustrates a two-dimensional display integrated into a magnetic resonance imaging magnet;
Fig. 6 shows an alternative view of the two-dimensional display of Fig. 5;
Fig. 7 illustrates a method of coupling the optical wave guide bundle to the two-dimensional display;
Fig. 8 illustrates a further method of coupling the optical wave guide bundle to the two-dimensional display; and
Fig. 9 illustrates a further method of coupling the optical wave guide bundle to the two-dimensional display.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

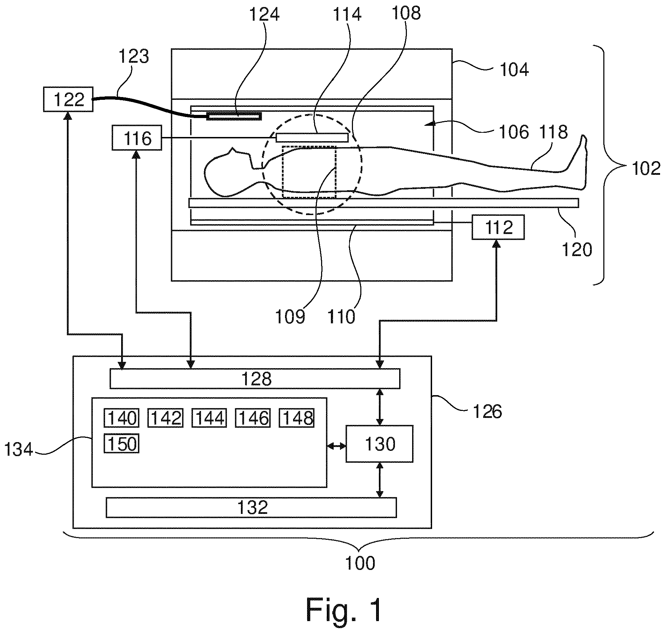

Fig. 1 illustrates an example of a magnetic resonance imaging system 100. The magnetic resonance imaging system 100 comprises a magnetic resonance imaging magnet assembly 102 and a computer system 126.

The magnetic resonance imaging magnet assembly 102 comprises a magnet 104. The magnet 104 is a superconducting cylindrical type magnet with a bore 106 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 106 of the cylindrical magnet 104 there is an imaging zone 108 where the magnetic field is strong and uniform enough to perform magnetic resonance imaging. A region of interest 109 is shown within the imaging zone 108. The magnetic resonance data is typically acquried for the region of interest. A subject 118 is shown as being supported by a subject support 120 such that at least a portion of the subject 118 is within the imaging zone 108 and the region of interest 109.

Within the bore 106 of the magnet there is also a set of magnetic field gradient coils 110 which is used for acquisition of preliminary magnetic resonance data to spatially encode magnetic spins within the imaging zone 108 of the magnet 104. The magnetic field gradient coils 110 connected to a magnetic field gradient coil power supply 112. The magnetic field gradient coils 110 are intended to be representative. Typically magnetic field gradient coils 110 contain three separate sets of coils for spatially encoding in three orthogonal spatial directions. A magnetic field gradient power supply supplies current to the magnetic field gradient coils. The current supplied to the magnetic field gradient coils 110 is controlled as a function of time and may be ramped or pulsed.

Adjacent to the imaging zone 108 is a radio-frequency coil 114 for manipulating the orientations of magnetic spins within the imaging zone 108 and for receiving radio transmissions from spins also within the imaging zone 108. The radio frequency antenna may contain multiple coil elements. The radio frequency antenna may also be referred to as a channel or antenna. The radio-frequency coil 114 is connected to a radio frequency transceiver 116. The radio-frequency coil 114 and radio frequency transceiver 116 may be replaced by separate transmit and receive coils and a separate transmitter and receiver. It is understood that the radio-frequency coil 114 and the radio frequency transceiver 116 are representative. The radio-frequency coil 114 is intended to also represent a dedicated transmit antenna and a dedicated receive antenna. Likewise the transceiver 116 may also represent a separate transmitter and receivers. The radio-frequency coil 114 may also have multiple receive/transmit elements and the radio frequency transceiver 116 may have multiple receive/transmit channels. For example if a parallel imaging technique such as SENSE is performed, the radio-frequency could 114 will have multiple coil elements.

Within the bore of the magnet 106 there can be seen that there is a two-dimensional display 124 that is attached to an interior surface. This for example may be attached to a magnet cover or embedded within it. The magnet cover is not shown in this Figure. There is an optical image generator 122 that is located out of the bore 106 of the magnet 104. Between the optical image generator 122 and the two-dimensional display 124 is an optical waveguide bundle 123. The optical waveguide bundle 123 couples the two-dimensional display 124 to the optical image generator 122. Details regarding the two-dimensional display 124 are discussed in later Figures.

The transceiver 116 and the gradient controller 112 are shown as being connected to a hardware interface 128 of a computer system 126. The computer system further comprises a processor 130 that is in communication with the hardware system 128, a memory 134, and a user interface 132. The memory 134 may be any combination of memory which is accessible to the processor 130. This may include such things as main memory, cached memory, and also non-volatile memory such as flash RAM, hard drives, or other storage devices. In some examples the memory 134 may be considered to be a non-transitory computer-readable medium.

The memory 134 is shown as containing machine-executable instructions 140. The machine-executable instructions 140 enable the processor 130 to control the operation and function of the magnetic resonance imaging system 100. The machine-executable instructions 140 may also enable the processor 130 to perform various data analysis and calculation functions. The computer memory 134 is further shown as containing pulse sequence commands 142.

The pulse sequence commands 142 enable the magnetic resonance imaging system to acquire magnetic resonance imaging data according to a magnetic resonance imaging protocol. The memory 134 is further shown as containing magnetic resonance imaging data 144 that has been acquired by controlling the magnetic resonance imaging system 100 with the pulse sequence commands 142. In the example shown in Fig. 1 there may be an optional subject motion detection system.

In this example the magnetic resonance imaging system 100 itself is the motion detection system. The pulse sequence commands 142 can be modified to also acquire navigator data 146. This may for example be useful for monitoring the breathing phase and/or heart phase of the subject 118. The memory 134 is shown as containing navigator data 146 that was acquired at the same time or interleaved with the acquisition of the magnetic resonance imaging data 144. The navigator data 146 may be the subject motion data and may be used to generate a motion feedback indicator 148. The motion feedback indicator 148 can be rendered on the two-dimensional display 124. This may be useful in the subject 118 controlling his or her position and/or breathing phase. The memory 134 is further shown as containing a magnetic resonance image 150 that was reconstructed from the magnetic resonance imaging data 144.

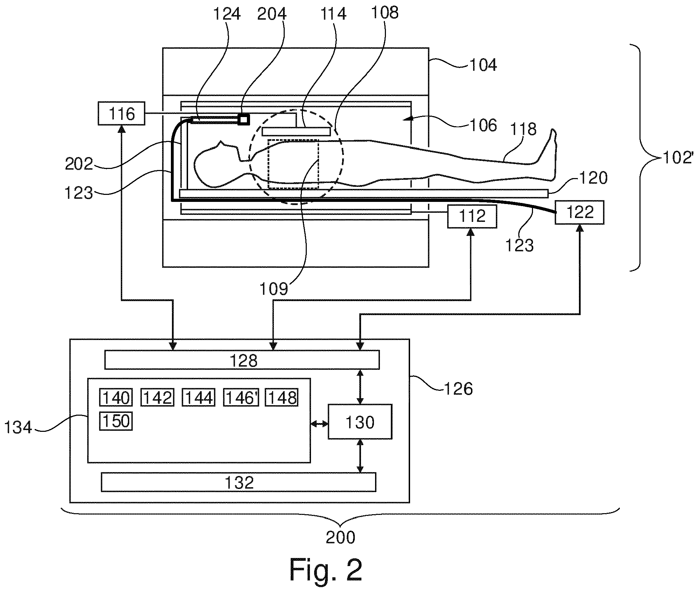

Fig. 2 illustrates a further example of a magnetic resonance imaging system 200. The magnetic resonance imaging system 200 is similar to the magnetic resonance imaging system 100 of Fig. 1 with several modifications. The magnetic resonance imaging magnet assembly 102' has been modified such that the optical image generator 122 is located on or near to the subject support 120 and the optical waveguide bundle 123 is routed through or is attached to the subject support 120. The two-dimensional display 124 is supported above the head of the subject 118 by a support arch 202. This holds the two-dimensional display 124 in a fixed position with relation to the subject 118 even if the subject support 120 is moved in and out of the bore 106 of the magnet 104. There is optionally a camera 204 attached to the support arch 202. The camera 204 may be used to acquire camera data 146' that in this case may be the subject motion data.

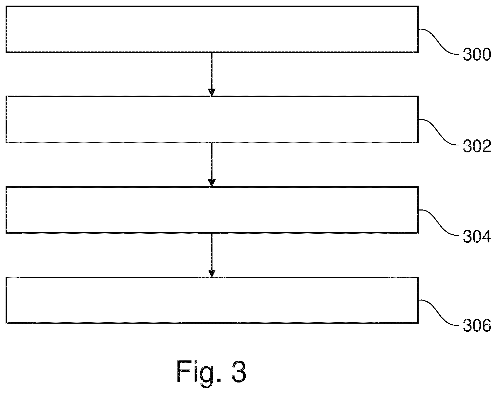

Fig. 3 shows a flowchart which illustrates a method of operating the magnetic resonance imaging system 100 of Fig. 1 or the magnetic resonance imaging system 200 of Fig. 2. First in step 300 the magnetic resonance imaging system 100, 200 is controlled with the pulse sequence commands 142. This causes the magnetic resonance imaging system 100, 200 to acquire the magnetic resonance imaging data 144. Next in step 302 the processor 130 controls the optical image generator 122 to generate the two-dimensional image during the acquisition of the magnetic resonance imaging data 144. For example, during the execution of the pulse sequence commands. The method then proceeds to step 304 which is optional. The subject motion detection system which in Fig. 1 is the magnetic resonance imaging system or in Fig. 2 the camera system 204, to acquire the subject motion data 146, 146' during the acquisition of the magnetic resonance imaging data 144. The method then proceeds optionally onto step 306 which control the optical image indicator to render the motion feedback indicator 148 as the two-dimensional image using the subject motion data to control the motion feedback indicator.

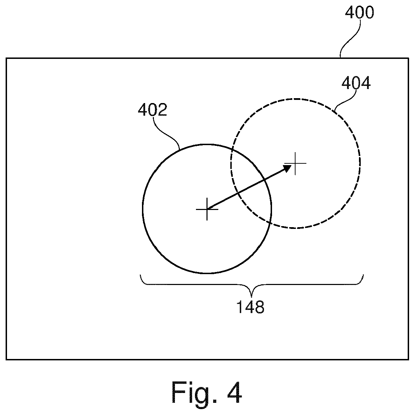

Fig. 4 illustrates an example of a two-dimensional image 400 which renders an example of a motion feedback indicator 148. In this example there are two circles, 402, 404. The first circle 402 represents an initial position of the subject and the second circle 404 represents a current position of the subject 404. The distance between the centers of the circle may for example be used to represent a change in a breathing phase or a more complex measurement of the subject's position may be mapped to a change in both the distance and/or orientation of the circles 402, 404.

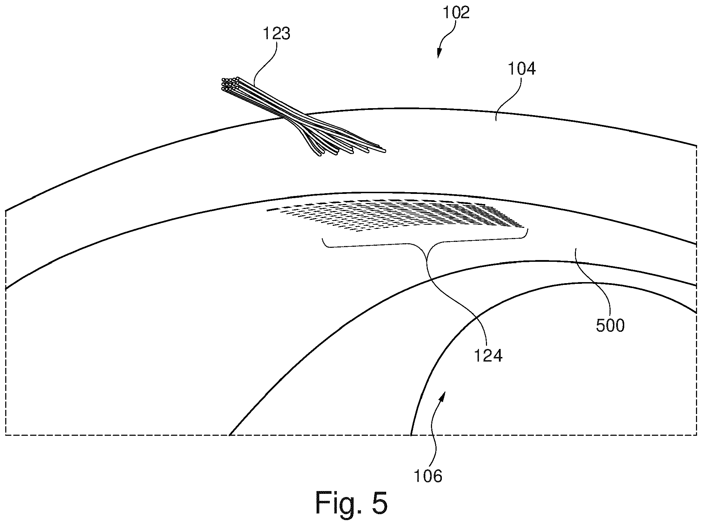

Examples may provide for a means to transfer an image (two-dimensional image) into the MRI bore through light guides in order to avoid any type of electromagnetic interference problems. This can be, for example, a bundle of glass fibers as shown in Fig. 5 below.

Fig. 5 illustrates an example of a two-dimensional display 124 such as would be present in the magnetic resonance imaging magnet assembly 102. Within the bore 106 of the magnet 104 the two-dimensional display 124 is shown as being integrated into a magnet cover 500. The optical waveguide bundle 123 is shown as going through the magnet cover 500 to the two-dimensional display 124. In this example the optical waveguide bundle 123 is a collection of fiber optic waveguides. In other examples the optical waveguide bundle 123 could be manufactured into or 3D printed into the magnet cover 500 or formed from lithographically structured foils.

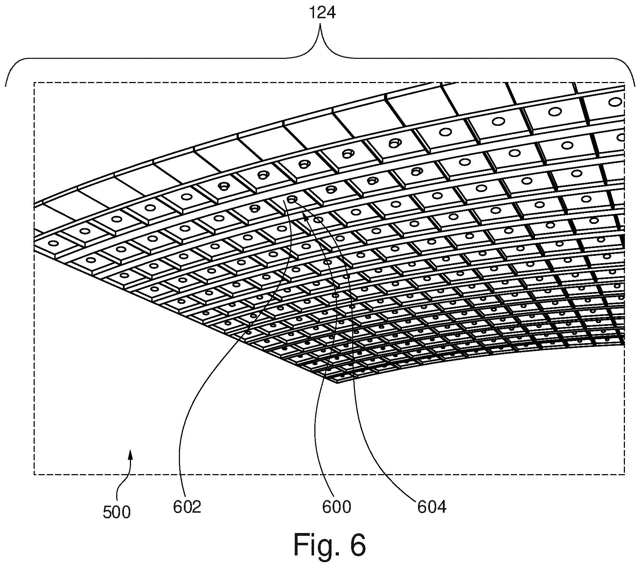

Fig. 6 shows an example of a two-dimensional display 124 in greater detail. In this example the two-dimensional display 124 is again mounted on the magnet cover 500, however the same display could be mounted on the support arch 202. The two-dimensional display 124 comprises a number of pixels 600. Each pixel 600 comprises a diffuser 602 and at least one optical waveguide 604 which is coupled to it. The diffuser 602 takes light from the optical waveguide 604 and makes it appear uniform across the surface of the pixel. This for example enables the subject to see and interpret the two-dimensional display 124 even when the angle of the subject with respect to the two-dimensional display 124 is not optimal.

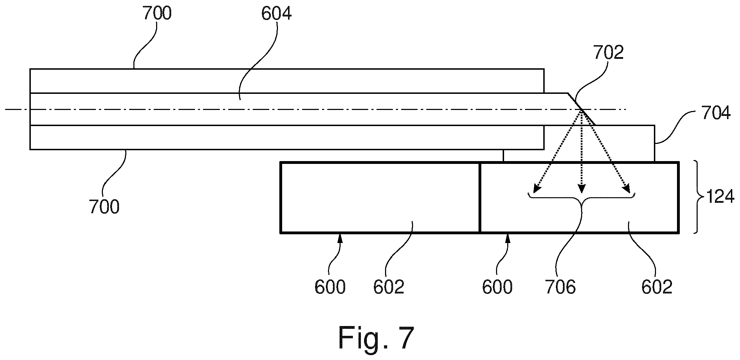

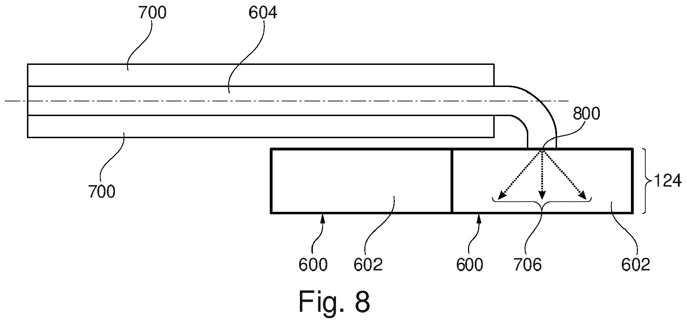

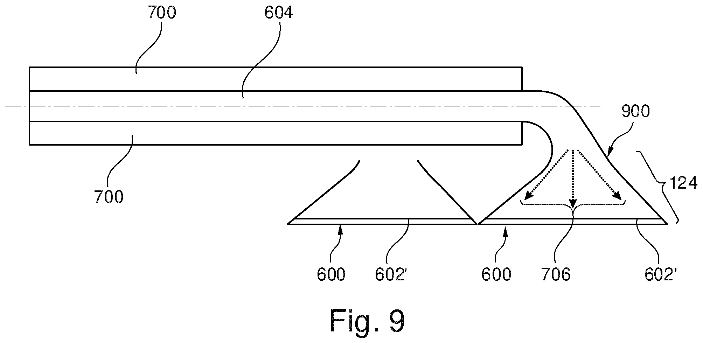

In the example of Fig. 5, one could project an image into one end of the fiber bundle. On the other end of the bundle, the fiber tips could make a bend and stick out into the MRI bore and are visible to the patient (see Fig. 9 below). Here, they can be arranged to form a two-dimensional display (see Fig. 7). The fiber diameter can be quite small, so in order to widen the pixels, one could terminate them with diffusor plates (Figs. 7 and 8). Figs. 7, 8 and 9 illustrate different ways of coupling the optical waveguide bundle to the two-dimensional display 124.

As an alternative to bending the fibers, one could also decouple the light by means of reflection at fiber ends honed and chamfered to 45°, This is illustrated in Fig. 7 below. Alternatively, instead of fibers, one could also use lithographically structured foil or a 3D printed waveguide structure.

Fig. 7 shows one example where an optical waveguide 604 has a reflective end 702. For example, the reflective end 702 could be polished and optionally coated with a mirror surface. This causes light 706 to be reflected through an optical coupler and then into the diffuser 602. The combination of the diffuser 602 and the coupler 704 forms one pixel 600 of the two-dimensional display 124. This may be replicated in other pixels 600. In this example the optical waveguide 604 was a fiber optic. Although a fiber optic is illustrated other types of waveguides such as a 3D-printed or polymer waveguide may also be used. The fiber optic 604 is shown as also optionally having a covering 700 for protecting the fiber 604. In some examples the optical coupler 604 is not used and the light 706 couples directly from the reflective end surface 702 to the diffuser 602.

Fig. 8 shows an alternative method of coupling light 706 into the diffusers 602 to form individual pixels 600. In the example the reflective end 702 is not used. Instead the fiber optic 604 is bent such that an optical coupling surface 800 abuts the diffuser 602 and the light 706 is then coupled.

Fig. 9 illustrates a further alternative for coupling the waveguides 604 to the two-dimensional display 124. In this example the waveguide 604 has a flaring structure 900 which transitions directly into the diffuser 602'. The structure illustrated in Fig. 9 may for example be representative of a system which is manufactured by three-dimensional printing. The diffuser 602' could for example be a different material that is printed and then the flaring structure 900 is printed and then finally, the optical waveguide 604 that connect with it. In another alternative the flaring structure 900 has its surface treated for example the end region may be frosted and this may be used to form the diffuser 602'.

While the invention has been illustrated and described in detail in the drawings and foregoing description, such illustration and description are to be considered illustrative or exemplary and not restrictive; the invention is not limited to the disclosed embodiments, without departing from the scope of the invention as defined by the appended claims.

### LIST OF REFERENCE NUMERALS

- 100: magnetic resonance imaging system
- 102: magnetic resonance imaging magnet assembly
- 102': magnetic resonance imaging magnet assembly
- 104: magnet
- 106: bore of magnet
- 108: imaging zone
- 109: region of interest
- 110: magnetic field gradient coils
- 112: magnetic field gradient coil power supply
- 114: radio-frequency coil
- 116: transceiver
- 118: subject
- 120: subject support
- 122: optical image generator
- 123: optical waveguide bundle
- 124: two-dimensional display
- 126: computer system
- 128: hardware interface
- 130: processor
- 132: user interface
- 134: computer memory
- 140: machine executable instructions
- 142: pulse sequence commands
- 144: magnetic resonance imaging data
- 146: navigator data (subject motion data)
- 146': camera data (subject motion data)
- 148: motion feedback indicator
- 150: magentic resonance image
- 200: magentic resonance imaging system
- 202: support arch
- 204: camera
- 300: acquire the magnetic resonance imaging data by controlling the magnetic resonance imaging system with the pulse sequence commands
- 302: control the optical image generator to generate the two-dimensional image during the acquisition of the magnetic resonance imaging data
- 304: control the subject motion detection system to acquire the subject motion data during the acquisition of the magnetic resonance imaging data
- 306: control the optical image indicator to render a motion feedback indicator within the two-dimensional image using the subject motion data
- 400: two dimensional image
- 402: initial position
- 404: current position
- 500: magnet cover
- 600: pixel
- 602: diffusor
- 602': diffusor
- 604: optical waveguide
- 700: optional covering
- 702: reflective end
- 704: optical coupler
- 706: light coupled to diffusor
- 800: optical coupling surface
- 900: flairing

## Claims

1. A magnetic resonance imaging magnet assembly (102, 102') configured for supporting a subject (118) within an imaging zone (108), wherein the magnetic resonance imaging magnet assembly comprises:
- a magnetic resonance imaging magnet (104), wherein the magnetic resonance imaging magnet is configured for generating a main magnetic field with the imaging zone;
- an optical image generator (122) configured for generating a two-dimensional image;
- an optical waveguide bundle (123) configured for coupling to the optical image generator;
- a two-dimensional display (124) comprising pixels (600), wherein each of the pixels comprises a diffusor (602, 602'), wherein the diffusor is a diffusor plate, wherein each of the pixels is optically coupled to at least one optical waveguide selected from the optical waveguide bundle, wherein the at least one optical waveguide of each of the pixels is configured for illuminating the diffusor, wherein the optical waveguide bundle and the two-dimensional display are configured for displaying the two-dimensional image.

2. The magnetic resonance imaging magnet assembly of claim 1, wherein the magnetic resonance imaging magnet assembly further comprises a subject support (120), wherein the optical waveguide bundle is integrated into the subject support.

3. The magnetic resonance imaging magnet assembly of claim 2, wherein the subject support comprises a support arch (202), wherein the two-dimensional display is attached to the support arch.

4. The magnetic resonance imaging magnet assembly of claim 1, wherein the magnetic resonance imaging magnet assembly comprises a gradient coil assembly (110), wherein the magnetic resonance imaging magnet assembly comprises a magnet cover (500) encasing the magnetic resonance imaging magnet and the gradient coil assembly, wherein the two-dimensional display is any one of the following: integrated into the magnet cover and attached to the magnet cover, and wherein the optical waveguide bundle is attached to the magnet cover, wherein the optical waveguide bundle is between the gradient coil assembly and the magnet cover.

5. The magnetic resonance imaging magnet assembly of claim 4, wherein the magnetic resonance imaging magnet is a cylindrical magnet with a bore (106) for receiving the subject, wherein the two-dimensional display is within the bore.

6. The magnetic resonance imaging magnet assembly of claim 5, wherein the optical image generator is attached to the magnetic resonance imaging magnet assembly, wherein the optical image generator is outside of bore.

7. The magnetic resonance imaging magnet assembly of any one of the preceding claims, wherein the optical waveguide bundle is a three-dimensional printed optical waveguide bundle or formed from lithographically structured foils.

8. The magnetic resonance imaging magnet assembly of any one of claims 1 through 6, wherein the optical waveguide bundle is formed from multiple optical fibers.

9. The magnetic resonance imaging assembly of any one of the preceding claims, wherein optical waveguides of the optical wave guide bundle are configured for any one of the following:
- forming an optical coupling surface (800) that abuts the diffusor of each voxel and
- forming the diffusor (602') on an end surface of the optical wave guide

10. The magnetic resonance imaging assembly of any one of claims 1 through 8, wherein the optical waveguides of the optical wave guide bundle comprise a reflective end surface (702), wherein the optical waveguides of the optical wave guide bundle are configured to couple to the diffusor using the reflective end surface.

11. A magnetic resonance imaging system comprising the magnetic resonance imaging magnet assembly of any one of the preceding claims, wherein the magnetic resonance imaging system further comprises:
- a memory (134) storing machine executable instructions (140) and pulse sequence commands (142) configured for controlling the magnetic resonance imaging system to acquire magnetic resonance imaging data (144);
- a processor (130) configured for controlling the magnetic resonance imaging system, wherein execution of the machine executable instructions causes the processor to:
- acquire (300) the magnetic resonance imaging data by controlling the magnetic resonance imaging system with the pulse sequence commands; and
- control (302) the optical image generator to generate the two-dimensional image during the acquisition of the magnetic resonance imaging data.

12. The magnetic resonance imaging system of claim 11, wherein the magnetic resonance imaging system further comprises a subject motion detection system (100, 204) configured for acquiring subject motion during the acquisition of the magnetic resonance imaging data, wherein execution of the machine executable instructions further cause the processor to:
- control (304) the subject motion detection system to acquire the subject motion data (146, 146') during the acquisition of the magnetic resonance imaging data; and
- control (306) the optical image indicator to render a motion feedback indicator (148) within the two-dimensional image using the subject motion data.

13. The magnetic resonance imaging system of claim 12, wherein the subject motion detection system comprises any one of the following: a body position sensor, a camera system (146'), a respiration tube, a respiration monitor belt, a magnetic resonance imaging navigator (146), and combinations thereof.

14. The magnetic resonance imaging system of claim 12 or 13, wherein the optical image indicator is configured for displaying any one of the following: a breath hold indicator, a breathing state of the subject, a body position of the subject, and combinations thereof.

15. The magnetic resonance imaging system of any one of claims 11 through 14, wherein execution of the machine executable instructions further causes the processor to control the optical image generator to perform any one of the following:
- render a chosen color pattern;
- render a chosen color gradient;
- render a chosen brightness gradient; and
- combinations thereof.

## Patentansprüche

1. Magnetanordnung (102, 102') zur Magnetresonanzbildgebung, konfiguriert zur Unterstützung eines Patienten (118) innerhalb einer Bildgebungszone (108), wobei die Magnetanordnung zur Magnetresonanzbildgebung Folgendes umfasst:
- einen Magnetresonanz-Bildgebungsmagneten (104), wobei der Magnetresonanz-Bildgebungsmagnet zum Erzeugen eines Hauptmagnetfeldes mit der Bildgebungszone konfiguriert ist;
- einen optischen Bildgenerator (122), zum Erzeugen eines zweidimensionalen Bildes;
- ein optisches Wellenleiterbündel (123), zur Kopplung mit dem optischen Bildgenerator zu koppeln;
- eine zweidimensionale Anzeige (124) mit Pixeln (600), wobei jedes der Pixel einen Diffusor (602, 602') umfasst, wobei der Diffusor eine Diffusorplatte ist, wobei jedes der Pixel optisch mit mindestens einem optischen Wellenleiter gekoppelt ist, der aus dem optischen Wellenleiterbündel ausgewählt ist, wobei der mindestens eine optische Wellenleiter jedes der Pixel zum Beleuchten des Diffusors konfiguriert ist, wobei das optische Wellenleiterbündel und die zwei-Dimensionale Anzeige zum Anzeigen des zweidimensionalen Bildes konfiguriert sind.

2. Magnetanordnung für die Magnetresonanzbildgebung nach Anspruch 1, wobei die Magnetanordnung für die Magnetresonanzbildgebung ferner eine Patientenhalterung (120) umfasst, wobei das Lichtwellenleiterbündel in die Patientenhalterung integriert ist.

3. Magnetanordnung für die Magnetresonanzbildgebung nach Anspruch 2, wobei die Patientenhalterung einen Trägerbogen (202) umfasst, wobei die zweidimensionale Anzeige an dem Trägerbogen befestigt ist.

4. Magnetanordnung für die Magnetresonanzbildgebung nach Anspruch 1, wobei die Magnetanordnung für die Magnetresonanzbildgebung eine Gradientenspulenanordnung (110) umfasst, wobei die Magnetanordnung für die Magnetresonanzbildgebung eine Magnetabdeckung (500) umfasst, die Magnetanordnung für die Magnetresonanzbildgebung und die Gradientenspulenanordnung umschließt, wobei diese in die Magnetabdeckung integriert und an der Magnetabdeckung befestigt ist, und wobei die zweidimensionale Anzeige eine der folgenden ist in die Magnetabdeckung integriert und an der Magnetabdeckung befestigt, und wobei das optische Wellenleiterbündel an Magnetabdeckung befestigt ist, wobei sich das optische Lichtwellenleiterbündel zwischen der Gradientenspulenanordnung und der Magnetabdeckung befindet.

5. Magnetanordnung für die Magnetresonanzbildgebung nach Anspruch 4, wobei die Magnet für die Magnetresonanztomographie ist ein zylindrischer Magnet mit einer Bohrung (106) zur Aufnahme des Patienten, wobei sich die zweidimensionale Anzeige innerhalb der Bohrung befindet.

6. Magnetanordnung für die Magnetresonanzbildgebung nach Anspruch 5, wobei die der optische Bildgenerator an der Magnetanordnung für die Magnetresonanzbildgebung befestigt ist, wobei sich der optische Bildgenerator außerhalb der Bohrung befindet.

7. Magnetanordnung für die Magnetresonanzbildgebung nach einem der vorhergehenden Ansprüche, wobei das optische Wellenleiterbündel ein dreidimensional gedrucktes optisches Wellenleiterbündel ist oder aus lithographisch strukturierten Folien gebildet ist.

8. Die Magnetanordnung für die Magnetresonanzbildgebung nach einem der Ansprüche 1 bis 6, wobei das optische Wellenleiterbündel aus mehreren optischen Fasern gebildet ist.

9. Die Magnetresonanzbildgebung nach einem der vorhergehenden Ansprüche, wobei die optischen Wellenleiter des optischen Wellenleiterbündels für einen der folgenden Punkte konfiguriert sind:
- Ausbilden einer optischen Kopplungsfläche (800), die an den Diffusor eines jeden Voxels angrenzt und
- Ausbilden des Diffusors (602') an einer Endfläche des optischen Wellenleiters

10. Die Magnetresonanzbildgebung nach einem der Ansprüche 1 bis 8, wobei die optischen Wellenleiter des optischen Wellenleiterbündels eine reflektierende Endoberfläche (702) aufweisen, wobei die optischen Wellenleiter des optischen Wellenleiterbündels so konfiguriert sind, dass sie unter Verwendung der reflektierenden Endoberfläche an den Diffusor koppeln.

11. Magnetresonanzbildgebungssystem, das die Magnetanordnung für die Magnetresonanzbildgebung nach einem der vorhergehenden Ansprüche umfasst, wobei das System für die Magnetresonanzbildgebung ferner Folgendes umfasst:
- einen Speicher (134), der maschinenausführbare Anweisungen (140) und Pulssequenzbefehle (142), die zur Steuerung des Magnetresonanzbildgebungssystems zur Erfassung von Magnetresonanzbildgebungsdaten (144) konfiguriert sind;
- einen Prozessor (130), der für die Steuerung des Magnetresonanzbildgebungssystems konfiguriert ist, wobei die Ausführung der maschinenausführbaren Anweisungen den Prozessor zu Folgendem veranlasst:
- Erfassung (300) der Magnetresonanzbildgebungsdaten durch Steuerung des Magnetresonanzbildgebungssystems mit den Pulssequenzbefehlen; und
- Steuerung (302) des optischen Bildgenerators zur Erzeugung des Zweidimensionalen Bildes während der Erfassung der Magnetresonanzbilddaten.

12. Magnetresonanzbildgebungssystem nach Anspruch 11, wobei das Magnetresonanzbildgebungssystem ferner ein Patientenbewegungserfassungssystem (100, 204) umfasst, das zum Erfassen von Patientenbewegungen während der Erfassung der Magnetresonanzbildgebungsdaten konfiguriert ist, wobei die Ausführung der maschinenausführbaren Anweisungen ferner bewirkt, den Prozessor zu Folgendem zu veranlassen:
- Steuerung (304) des Systems zur Erfassung von Patientenbewegungen zur Erfassung der Patienten Bewegungsdaten (146, 146') während der Erfassung der Magnetresonanzbilddaten; und
- Steuern (306) der optischen Bildanzeige, um eine Bewegungsrückmeldungsanzeige (148) innerhalb des zweidimensionalen Bildes unter Verwendung der Patientenbewegungsdaten zu liefern.

13. Magnetresonanzbildgebungssystem nach Anspruch 12, wobei das Patientenbewegungserfassungssystem eines der folgenden Elemente umfasst: einen Körperpositionssensor, ein Kamerasystem (146'), eine Atmungsröhre, einen Atmungsüberwachungsgürtel, einen Magnetresonanzbildgebungsnavigator (146) und Kombinationen davon.

14. Magnetresonanzbildgebungssystem nach Anspruch 12 oder 13, wobei der optische Bildindikator so konfiguriert ist, dass er eines der folgenden Elemente anzeigt: einen Atemstillstandsindikator, einen Atmungszustand der Testperson, eine Körperposition der Testperson und Kombinationen davon.

15. Das Magnetresonanzbildgebungssystem nach einem der Ansprüche 11 bis 14, wobei die Ausführung der maschinenausführbaren Anweisungen den Prozessor ferner veranlasst, den optischen Bildgenerator zu steuern, um eines der folgenden Verfahren durchzuführen:
- ein ausgewähltes Farbmuster wiedergeben;
- einen gewählten Farbverlauf wiedergeben;
- einen gewählten Helligkeitsgradienten wiedergeben; und
- Kombinationen davon.

## Revendications

1. Assemblage magnétique d'imagerie par résonance magnétique (102, 102') configuré pour soutenir un sujet (118) dans une zone d'imagerie (108), dans lequel l'assemblage magnétique d'imagerie par résonance magnétique comprend:
- un aimant d'imagerie par résonance magnétique (104), dans lequel l'aimant d'imagerie par résonance magnétique est configuré pour générer un champ magnétique principal avec la zone d'imagerie;
- un générateur d'images optiques (122) configuré pour générer une bidimensionnelle image;
- un faisceau de guides d'ondes optiques (123) configuré pour être couplé au générateur d'images optiques ;
- un affichage bidimensionnel (124) comprenant des pixels (600), dans lequel chacun des pixels comprend un diffuseur (602, 602'), dans lequel le diffuseur est une plaque de diffusion, dans lequel chacun des pixels est optiquement couplé à au moins un guide d'ondes optiques sélectionné dans le faisceau de guides d'ondes optiques, dans lequel le au moins un guide d'ondes optiques de chacun des pixels est configuré pour éclairer le diffuseur, dans lequel le faisceau de guides d'ondes optiques et l'écran bi-dimensionnel sont configurés pour afficher l'image bidimensionnelle.

2. Assemblage magnétique d'imagerie par résonance magnétique selon la revendication 1, dans lequel l'assemblage magnétique d'imagerie par résonance magnétique comprend en outre un support de sujet (120), dans lequel le faisceau de guides d'ondes optiques est intégré au support du sujet.

3. Assemblage magnétique d'imagerie par résonance magnétique selon la revendication 2, dans lequel le support du sujet comprend une arche de support (202), dans laquelle l'affichage bidimensionnel est fixé à l'arche de support.

4. Aimant d'imagerie par résonance magnétique selon la revendication 1, dans lequel l'aimant d'imagerie par résonance magnétique comprend une bobine de gradient (110), dans lequel l'assemblage magnétique d'imagerie par résonance magnétique comprend un couvercle d'aimant (500) enveloppant l'aimant d'imagerie par résonance magnétique et l'ensemble de bobine de gradient, dans lequel l'affichage bidimensionnel est l'un des suivants: intégré dans le couvercle d'aimant et fixé au couvercle d'aimant, et dans lequel le faisceau de guides d'ondes optiques est fixé au couvercle d'aimant, dans lequel le faisceau de guides d'ondes optiques se trouve entre l'ensemble de bobine de gradient et le couvercle d'aimant.

5. Assemblage magnétique d'imagerie par résonance magnétique selon la revendication 4, dans lequel l'aimant d'imagerie par résonance magnétique est un aimant cylindrique avec un alésage (106) pour recevoir le sujet, dans lequel l'affichage bidimensionnel se trouve à l'intérieur de l'alésage.

6. Assemblage magnétique d'imagerie par résonance magnétique selon la revendication 5, dans lequel le générateur d'images optiques est fixé à l'assemblage magnétique d'imagerie par résonance magnétique, dans lequel le générateur d'images optiques est à l'extérieur de l'alésage.

7. Assemblage magnétique d'imagerie par résonance magnétique selon l'une des revendications précédentes, dans lequel le faisceau de guides d'ondes optiques est un faisceau de guides d'ondes optiques imprimé en trois dimensions ou formé à partir de feuilles structurées par lithographie.

8. Assemblage magnétique d'imagerie par résonance magnétique selon l'une des revendications 1 à 6, dans lequel le faisceau de guides d'ondes optiques est formé de plusieurs fibres optiques.

9. Assemblage d'imagerie par résonance magnétique selon l'une des revendications précédentes, dans lequel les guides d'ondes optiques du faisceau de guides d'ondes optiques sont configurés pour l'une des opérations suivantes :
- former une surface de couplage optique (800) qui bute sur le diffuseur de chaque voxel et
- former le diffuseur (602') sur une surface d'extrémité du guide d'ondes optiques

10. Assemblage d'imagerie par résonance magnétique selon l'une des revendications 1 à 8, dans lequel les guides d'ondes optiques du faisceau de guides d'ondes optiques comprennent une surface d'extrémité réfléchissante (702), dans lequel les guides d'ondes optiques du faisceau de guides d'ondes optiques sont configurés pour se coupler au diffuseur à l'aide de la surface d'extrémité réfléchissante.

11. Système d'imagerie par résonance magnétique comprenant l'assemblage magnétique d'imagerie par résonance magnétique selon l'une des revendications précédentes, dans lequel le système d'imagerie par résonance magnétique comprend en outre:
- une mémoire (134) stockant des instructions exécutables par machine (140) et des commandes de séquence d'impulsions (142) configurées pour commander le système d'imagerie par résonance magnétique afin d'acquérir des données d'imagerie par résonance magnétique (144);
- un processeur (130) configuré pour commander le système d'imagerie par résonance magnétique, dans lequel l'exécution des instructions exécutables par machine amène le processeur à:
- acquérir (300) les données d'imagerie par résonance magnétique en commandant le système d'imagerie par résonance magnétique à l'aide des commandes de séquence d'impulsions; et
- commander (302) le générateur d'images optiques pour générer l'image bi-dimensionnelle pendant l'acquisition des données d'imagerie par résonance magnétique.

12. Système d'imagerie par résonance magnétique selon la revendication 11, dans lequel le système d'imagerie par résonance magnétique comprend en outre un système de détection du mouvement du sujet (100, 204) configuré pour acquérir le mouvement du sujet pendant l'acquisition des données d'imagerie par résonance magnétique, dans lequel l'exécution des instructions exécutables par machine amène en outre le processeur à:
- commander (304) le système de détection du mouvement du sujet pour acquérir les données de mouvement du sujet (146, 146') pendant l'acquisition des données d'imagerie par résonance magnétique; et
- commander (306) l'indicateur d'image optique pour rendre un indicateur de retour de mouvement (148) dans l'image bidimensionnelle à l'aide des données de mouvement du sujet.

13. Système d'imagerie par résonance magnétique selon la revendication 12, dans lequel le système de détection du mouvement du sujet comprend l'un des éléments suivants, un système de caméra (146'), un tube respiratoire, une ceinture de surveillance respiratoire, un navigateur d'imagerie par résonance magnétique (146), et des combinaisons de ceux-ci.

14. Système d'imagerie par résonance magnétique selon la revendication 12 ou 13, dans lequel l'indicateur d'image optique est configuré pour afficher l'un des éléments suivants: un indicateur d'arrêt de la respiration, un état respiratoire du sujet, une position corporelle du sujet, et des combinaisons de ces éléments.

15. Système d'imagerie par résonance magnétique selon l'une des revendications 11 à 14, dans lequel l'exécution des instructions exécutables par la machine amène en outre le processeur à commander le générateur d'images optiques pour effectuer l'une des opérations suivantes :
- rendre un motif de couleur choisi;
- rendre un gradient de couleur choisi;
- rendre un gradient de luminosité choisi; et
- des combinaisons de ces éléments.
